# EUROPÄISCHE PATENTSCHRIFT

(11) **EP 1 247 083 B1**
(45) Veröffentlichungstag und Bekanntmachung des Hinweises auf die Patenterteilung: **01.02.2006**
(21) Anmeldenummer: 00987320.9
(22) Anmeldetag: 29.11.2000
(51) Int. Cl.: G01M 11/00

(54) **VORRICHTUNG ZUM MESSTECHNISCHEN ERFASSEN VON TRANSMISSIONSVERLUSTEN**
DEVICE FOR DETECTING TRANSMISSION LOSSES BY MEANS OF MEASUREMENTS
DISPOSITIF POUR LA SAISIE METROLOGIQUE DE PERTES DE TRANSMISSION

(30) Priorität: 14.01.2000 DE 10001289
(43) Veröffentlichungstag der Anmeldung: 09.10.2002
(73) Patentinhaber: Ferton Holding SA, 2800 Delémont (CH)
(72) Erfinder: HIRT, Joachim, 78465 Konstanz (DE); GLENZER, Thomas, CH-8280 Kreuzlingen (CH); KAMP, Armin, 78315 Radofzell (DE)
(74) Vertreter: Dallmeyer, Georg
(86) Internationale Anmeldenummer: PCT/EP2000/011930
(87) Internationale Veröffentlichungsnummer: WO 2001/051905

(56) Entgegenhaltungen:
- GB-A- 1 532 423
- US-A- 5 115 126
- US-A- 5 268 732

## Beschreibung

Die Erfindung betrifft eine Vorrichtung zum meßtechnischen Erfassen von Transmissionsverlusten von optischen Lichtleiteinrichtungen eines Endoskopes nach dem Oberbegriff des Anspruchs 1.

Derartige Lichtleiteinrichtungen bestehen beispielsweise aus den Lichtleitkabeln für Endoskope und den Beleuchtungsleitern im Endoskop. Die endoskopischen Instrumente werden für die minimalinvasive Chirurgie benötigt. Ein Endoskop besteht üblicherweise aus einer bilderzeugenden Optik, die ein Objekt aus dem Körperinneren dem Chirurg über ein Okular oder eine daran angeschlossene Videokamera sichtbar macht. Zusätzlich enthält das Endoskop eine optische Beleuchtungseinrichtung, mit der die Körperhöhle beleuchtet wird. Für die Beleuchtung des Operationsgebietes wird üblicherweise eine externe Lichtquelle verwendet, die mit dem Endoskop über ein flexibles Lichtleitkabel verbunden ist. Das Licht wird im Endoskop über einen optischen Eingang eingekoppelt und über ein Bündel von Lichtleitfasern an den optischen Ausgang an dem distalen Ende des Endoskops weitergeleitet.

Bei der Überprüfung von Endoskopen und zugehörigen Lichtleitkabeln hat sich häufig gezeigt, daß die Lichtleiteinrichtungen hohe Transmissionsverluste aufweisen. Durch mechanische Beanspruchung wie Biegen und Aufwickeln des Lichtleitkabels, Zugspannungen durch kräftiges Ziehen an einem Lichtleitkabel, Biegen des Endoskopschaftes und thermische Spannungen beim Heißdampf-Sterilisieren können einzelne Fasern der Lichtleiteinrichtungen brechen. Häufig befinden sich auch Ablagerungen auf den optischen Ein- und Austrittsflächen der optischen Eingänge und Ausgänge. Diese Fehler führen in der Regel nicht zu einem sofortige Ausfall der gesamten Beleuchtungseinrichtungen, sondern zu einer fortschreitenden Verschlechterung der Transmission. Wegen des allmählichen Überganges zu höheren Transmissionsverlusten wird die Verschlechterung der Beleuchtungsintensität oft zu spät bemerkt. Stellt der. Chirurg während eines Eingriffes fest, daß das eingesetzte Endoskop den Eingriffsort nicht ausreichend beleuchten kann, so muß er die Operation meist abbrechen, da ihm kein weiteres sterilisiertes Endoskop zur Verfügung steht.

Es wäre daher erforderlich, die endoskopischen Instrumente vor ihrem Einsatz zu prüfen.

Geräte zur Bestimmung von verschiedenen photometrischer Meßgrößen sind bekannt. Diese Instrumente verwenden einen optischen Sensor um das Licht zu erfassen, das von der Lichtquelle über die Lichtleiteinrichtungen austritt.

Aus der DE 35 15 612 A ist ein Lichtquellengerät für ein Endoskop mit einer Prüfeinrichtung für die Lichtquelle beschrieben. Eine Überprüfung der Lichtleitkabel oder eines Endoskops ist nicht vorgesehen.

In der DE 43 25 671 A ist ein Verfahren bzw. eine Vorrichtung zur Messung der Dämpfung in Lichtwellenleitern unter Verwendung eines gepulsten Lichtsenders beschrieben. Dabei wird das Sendesignal mit einer Pulsfrequenzmodulation belegt, die Schwankungen in der Ausgangsleistung beseitigt. Diese Meßmethode ist sehr genau, aber für die schnelle Prüfung von Lichtleiteinrichtungen in Operationssälen zu aufwendig.

Aus der EP 0 416 408 A ist eine Prüfungseinrichtung bekannt, bei der die Lichtquelle der endoskopischen Einrichtung im Strahlengang einen Strahlenteiler aufweist. Ein Teil des Lichtstrahls wird auf einen ersten Sensor geleitet, während der andere Teil über ein zu prüfendes Lichtleitkabel auf einen zweiten Sensor geleitet wird. Durch Vergleich der Meßwerte des ersten und des zweiten Sensors wird der Transmissionsgrad ermittelt. Nachteilig ist dabei, daß zwei Lichtsensoren erforderlich sind, die eine unterschiedliche Empfindlichkeit aufweisen können und daher kalibriert werden müssen. Auch die Aufteilung des Lichtstrahls mit Hilfe eines Strahlenteilers macht eine sorgfältige Kalibration erforderlich. Die Prüfeinrichtung verfügt nicht über eine eigene Lichtquelle und ist daher von der Lichtquelle der endoskopischen Einrichtung abhängig, so daß zusätzlich Helligkeitsgrenzwerte festgelegt sein müssen, um die Lichtintensität der Lichtquelle zu beurteilen. Nachteilig ist dabei, daß die Prüfeinrichtung in eine bestehende Endoskopausrüstung integriert ist und nicht generell zur Überprüfung von Lichtleiteinrichtungen eines Endoskopes einsetzbar ist. Insbesondere läßt die bekannte Einrichtung nur die Prüfung von Lichtleitkabeln oder der Lampe der Lichtquelle einer bestimmten Einrichtung zu, nicht jedoch die Überprüfung des Endoskopes selbst.

In D1 wird ein Lichtleiter mit beiden Enden an eine Kugelförmige Kammer angeschlossen und das durch den Lichtleiter geleitete Licht gemessen.

Der Erfindung liegt daher die Aufgabe zugrunde, eine Vorrichtung zur meßtechnischen Erfassung von Transmissionsverlusten von optischen Lichtleiteinrichtungen eines Endoskopes zu schaffen, die eine schnelle Prüfung unterschiedlicher Lichtleiteinrichtungen unabhängig von der Lichtquelle der endoskopischen Einrichtung ermöglicht.

Zur Lösung dieser Aufgabe dienen die Merkmale des Anspruchs 1.

Die Erfindung sieht dem vorteilhafter Weise vor, daß der mindestens eine Lichtsensor in einer Kammer angeordnet ist, daß der optische Ausgang der zu prüfenden Lichtleiteinrichtung durch eine Öffnung der Kammer in die Kammer einführbar ist und daß die die Kammer begrenzende Innenfläche diffus das aus dem optischen Ausgang der zu prüfenden Einrichtung austretende Licht reflektiert.

Die Anordnung des Lichtsensors in einer diffus reflektierenden Kammer hat den Vorteil, daß das aus der zu prüfenden Einrichtung austretende Licht weitestgehend unabhängig von seiner Richtung und seinem Eintrittsquerschnitt, erfaßt werden kann. Es ist daher möglich, unterschiedliche Lichtleiteinrichtungen unterschiedlicher Hersteller auf ihre Transmissionsverluste zu prüfen. Selbst die Lichtleiteinrichtungen von Endoskopen sind ohne großen Aufwand und mit hoher Reproduzierbarkeit der Meßwerte prüfbar. Dabei wird der Eintritt von Fremdlicht an der Öffnung der Kammer beispielsweise mit einer oder mehreren flexiblen Dichtungen zuverlässig verhindert. Die erfindungsgemäße Vorrichtung ist unabhängig von den endoskopischen Einrichtungen universell einsetzbar und eignet sich.zur schnellen Überprüfung von Lichtleiteinrichtungen vor einer Operation. Insbesondere ist es nicht mehr erforderlich, ein Endoskop zur Überprüfung seiner Transmissionsverluste an den Hersteller zurückzusenden.

Vorzugsweise ist vorgesehen, daß eine von der zu prüfenden Einrichtung unabhängige separate Lichtquelle eine vorbestimmte Lichtintensität in den optischen Eingang der zu prüfenden Lichtleiteinrichtung einkoppelt. Der Einsatz einer separaten Lichtquelle hat den Vorteil, daß eine definierte vorbestimmte Lichtintensität zur Prüfung verwendet werden kann, ohne daß für jede Prüfung eine erneute Kalibration von Referenzwerten erfolgen muß. Endoskopische Einrichtungen verwenden in der Regel Halogenlampen oder Gasentladungslampen. Diese Lichtquellen besitzen eine hohe Strahlungsleistung und sind meistens geregelt, so daß die Einstellung einer reproduzierbaren konstanten Lichtintensität schwierig ist. Auch unterliegen diese Lichtquellen einem Alterungsprozeß, so daß auch fest eingestellte Regelwerte einer Kalibrierung bedürfen. Im Gegensatz hierzu kann bei Einsatz einer eigenen Prüflichtquelle eine konstante Lichtintensität vorausgesetzt werden, die eine ständige Kalibrierung nicht benötigt.

Besonders vorteilhaft ist dabei die Verwendung einer Leuchtdiode als Lichtquelle. Da die Messung der Transmissionsverluste keine hohen Lichtintensitäten erfordert, ist der Einsatz einer Leuchtdiode möglich. Sie ist preisgünstig und läßt sich mit einer Konstantstrom-Ansteuerung auf eine ausreichende, in hohem Maße reproduzierbare Lichtintensität einstellen. Eine Kalibrierung kann daher entfallen, da für die nur für Prüfzwecke verwendete Lichtquelle von einem fest eingestellten und gespeicherten Referenzwert ausgegangen werden kann.

Eine bevorzugte Weiterbildung der Erfindung sieht vor, daß die Lichtquelle in einem Anschlußteil angeordnet ist, das an standardisierte Anschlüsse des optischen Eingangs der zu prüfenden Einrichtung angepaßt ist. Das an die standardisierten Anschlüsse angepaßte Anschlußteil erlaubt ein schnelles Anschließen der Lichtquelle an der Eingangsseite der lichtleitenden Einrichtung und einen sicheren Sitz an der zu prüfenden Einrichtung.

Bei einer bevorzugten Weiterbildung ist vorgesehen, daß die Innenfläche der Kammer kugelförmig ist. Für die Genauigkeit der Messung ist es wichtig, daß das gesamte transmittierte Licht erfaßt und ausgewertet wird. Endoskope beleuchten beispielsweise den Beobachtungsort je nach Ausführungsform und Einsatzzweck unter unterschiedlich großen Öffnungswinkeln. Auch kann der Lichtaustritt der Lichtleiteinrichtung gegenüber der Instrumentenachse abgewinkelt sein. Die von einer kugelförmigen Innenfläche der gebildete Kammer ist in der Lage, unabhängig von dem Abstrahlwinkel und dem Durchmesser des Lichtaustritts das gesamte aus dem optischen Ausgang austretende Licht diffus zu reflektieren, so daß der in der Kammer angeordnete mindestens ein Lichtsensor das gesamte Licht in hohem Maße reproduzierbar messen kann. Hierzu ist die Innenfläche der Hohlkugel weiß-diffus hochreflektierend beschichtet, oder besteht aus einem Material mit entsprechenden Eigenschaften. Das Licht wird durch diese Beschichtung mehrfach reflektiert, wodurch die Beleuchtungsintensität in der Kammer, in der sich der Lichtsensor befindet homogenisiert wird.

Vor dem Lichtsensor kann eine Diffusorscheibe angeordnet sein, die verhindert, daß direkt aus dem Lichtausgang der zu prüfenden Lichtleiteinrichtung austretendes Licht unmittelbar auf den Lichtsensor fallen kann. Die kugelförmige Kammer gewährleistet somit eine Unempfindlichkeit der Messung hinsichtlich der Austrittsrichtung des Lichtes an dem optischen Ausgang der zu prüfenden Lichtleiteinrichtung und auch hinsichtlich der exakten Lage des optischen Ausgangs innerhalb der Kammer.

Als Lichtsensor wird eine Photodiode verwendet.

In der Auswerteeinrichtung kann ein Referenzwert für die Lichtintensität der Lichtquelle gespeichert sein, wobei die Auswerteeinrichtung als Maß für die Transmissionsverluste die von dem Lichtsensor gemessene Lichtintensität ins Verhältnis zum Referenzwert setzt und beispielsweise als Prozentwert anzeigt.

Der Referenzwert für die Lichtquelle kann auch durch direkten Anschluß der Lichtquelle an die Öffnung der Kammer und Messung der Referenzintensität in die Auswerteeinrichtung eingespeichert werden.

Eine derartige Vorgehensweise ist beispielsweise bei einem Austausch der Lichtquelle durch eine andere oder neue Lichtquelle sinnvoll.

Zusätzlich können für bestimmte zu prüfende endoskopische Einrichtungen Referenzwerte für Transmissionsverluste in der Auswerteeinrichtung gespeichert sein, so daß die Anzeigeeinrichtung ein eventuelles Überschreiten vorgegebener Mindesttransmissionswerte anzeigen kann.

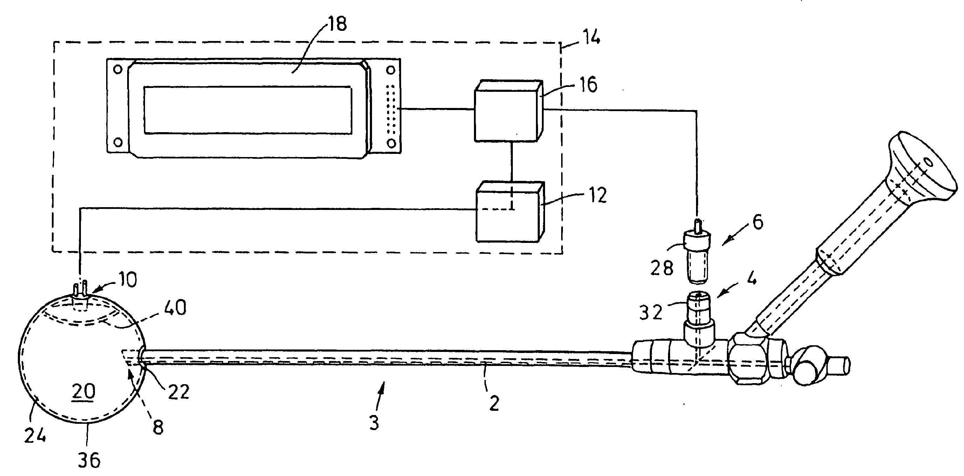

Im Folgenden wird unter Bezugnahme auf die Zeichnung ein Ausführungsbeispiel der Erfindung näher erläutert. Die einzige Figur zeigt die erfindungsgemäße Vorrichtung zur meßtechnischen Erfassen des Transmissionsverlustes einer endoskopischen Einrichtung.

Die in der einzigen Figur dargestellte Vorrichtung zum meßtechnischen Erfassen von Transmissionsverlusten von optischen Lichtleiteinrichtungen 2 eines Endoskopes 3 besteht im einzelnen aus einer Auswerteeinrichtung 14, einer von der zu untersuchenden Einrichtung 2 unabhängigen Lichtquelle 6 und einem in einer Hohlkugel 36 angeordneten Lichtsensor 10.

Die Auswerteeinrichtung 14 weist eine Anzeigeeinrichtung 18 zur Anzeige des Meßergebnisses auf sowie eine elektronische Schaltung 16, die einerseits eine Konstantstromversorgung für eine aus einer Photodiode bestehenden Lichtquelle 6 und andererseits eine Speichereinrichtung für Referenzwerte hinsichtlich der verwendeten Lichtquelle und hinsichtlich der zu prüfenden Lichtleiteinrichtungen enthält. Ferner weist die elektrische Schaltung 16 einen Eingang für das Ausgangssignal des mindestens einen Lichtsensors 10, das über einem Verstärker 12 der elektronischen Schaltung 16 zugeführt wird. Die elektronische Schaltung 16 setzt das verstärkte Meßsignal des Lichtsensors 10 ins Verhältnis zu einem vorbestimmten Referenzwert und zeigt das Meßergebnis auf der Anzeigeeinrichtung 18 an. Der Transmissionsverlust kann dabei beispielsweise als Prozentzahl angezeigt werden. Damit erhält der Anwender ein Maß für den Transmissionverlust der zu prüfenden Lichtleiteinrichtungen 2. Diese Lichtleiteinrichtungen 2 bestehen beispielsweise aus Lichtleitkabeln oder aus in dem Endoskop enthaltenen Lichtleitfasern. Wenn für bestimmte Lichtleiteinrichtungen 2 Referenzwerte für Transmissonsverluste gespeichert sind, ist die Anzeige des gemessenen Transmissionsverlustes in Relation zu einem Neu- oder Referenzinstrument möglich.

Die Lichtquelle 6 besteht vorzugsweise aus einer Leuchtdiode, die in einem Anschlußteil 28 angeordnet ist. Das Anschlußteil 28 paßt beispielsweise auf den standardisierten Anschluß 32 des Endoskopes 3 für Lichtleitkabel.

Das Anschlußteil 28 paßt desweiteren auch an den Einganganschluß der Lichtleitkabel. Somit ist es möglich, entweder Endoskope oder Lichtleitkabel oder auch andere lichtübertragende Lichtleiteinrichtungen zu untersuchen, wobei evtl. ein Adaptionsteil für den Anschluß der Lichtquelle 6 erforderlich ist.

Wie aus der Figur ersichtlich ist, ist das distale Ende des Endoskops 3 in eine Kammer 20 einer Hohlkugel 24 durch eine Öffnung 22 eingeführt. Die Öffnung ist in geeigneter Weise zum Beispiel durch eine oder mehrere in der Figur nicht dargestellten Gummidichtungen gegenüber Umgebungslicht abgedichtet.

Die Hohlkugel ist an ihrer Innenfläche 24 matt-weiß hochreflektierend beschichtet, oder besteht aus einem matt-weiß hochreflektierenden Material, z.B. Tefflon, so daß das aus dem distalen Ende des Endoskops 3 an dem optischen Ausgang 8 austretende Licht an der Innenfläche 24 der Hohlkugel 36 diffus reflektiert wird.

Das in der Kammer 20 reflektierte und diffus gestreute homogenisierte Licht wird von dem Lichtsensor 10 gemessen, wobei eine Diffusorscheibe 40 vor dem aus einer Photodiode bestehenden Lichtsensor 10 angeordnet sein kann, um eine Beaufschlagung des Lichtsensors 10 mit Direktlicht aus dem optischen Ausgang der Lichtleiteinrichtung 2 zu verhindern. Dies ist insbesondere dann erforderlich, wenn beispielsweise die Beleuchtungseinrichtung des Endoskopes 3 am optischen Ausgang 8 einen breiten Lichtkegel erzeugt.

Der Lichtsensor 10 ist in dem Ausführungsbeispiel der einzigen Figur unter einem Winkel von 90° zu der Öffnung 22 der Kammer 20 angeordnet. Die Öffnung 22 kann selbstverständlich unter einem Winkel von weniger als 90° zu der optischen Achse des Lichtsensors 10 angeordnet sein. Mit Hilfe der an ihrer Innenfläche 24 diffus reflektierenden Hohlkugel 36 und der Diffusorscheibe 40 kann der Lichtsensor 10 unabhängig von dem Eintrittswinkel des Lichtes an der Öffnung 22 und unter Homogenisierung des eintretenden Lichtes eine in hohem Maße und Genauigkeit reproduzierbare Messung durchführen. Mit Hilfe der Auswerteeinrichtung 14 kann dann der Transmissionsverlust zwischen dem optischen Eingang 4 und dem optischen Ausgang 8 in einfacher Weise mit geringstem Zeitaufwand bestimmt werden.

## Patentansprüche

1. Vorrichtung zum messtechnischen Erfassen von Transmissionsverlusten von optischen Lichtleiteinrichtungen (2) mit einem optischen Eingang (4) der zu prüfenden Lichtleiteinrichtung (2), durch den Licht einer Lichtquelle (6) zu einem optischen Ausgang (8) übertragen wird, mit mindestens einem in einer Kammer (20) angeordneten Lichtsensor (10), der die aus dem optischen Ausgang (8) der zu prüfenden Lichtleiteinrichtung (2) austretende und in der Kammer (20) diffus reflektierte Lichtintensität misst und einer Auswerteeinrichtung (14) für das Messsignal des mindestens einen Lichtsensors (10) mit einer Anzeigeeinrichtung (18) zur Anzeige des Messergebnisses,
**dadurch gekennzeichnet,**
**dass** die zu prüfende Lichtleiteinrichtung aus einem Endoskop besteht,
**dass** der optische Eingang (4) der zu prüfenden Lichtleiteinrichtung (2) sich an der Eingangsseite der in dem Endoskop (3) enthaltenen Lichtleiteinrichtung (2) befindet,
**dass** der optische Ausgang (8) des Endoskops (3) durch eine Öffnung (22) der Kammer (20) in die Kammer (20) einführbar ist, und
**dass** eine von der zu prüfenden in dem Endoskop (3) befindlichen Lichtleiteinrichtung (2) unabhängige separate Lichtquelle (6) eine vorbestimmte Lichtintensität in den optischen Eingang (4) des Endoskops (3) einkoppelt.

2. Vorrichtung nach Anspruch 1, **dadurch gekennzeichnet, daß** die Lichtquelle (6) aus einer Leuchtdiode besteht.

3. Vorrichtung nach einem der Ansprüche 1 bis 2, **dadurch gekennzeichnet, daß** die Lichtquelle (6) in einem Anschlußteil (28) angeordnet ist, das an den Anschluß (32) des optischen Eingangs der zu prüfenden Lichtleiteinrichtung (2) angepaßt ist.

4. Vorrichtung nach einem der Ansprüche 1 bis 3, **dadurch gekennzeichnet, daß** die Kammer (20) eine kugelförmige Innenfläche (24) aufweist.

5. Vorrichtung nach einem der Ansprüche 1 bis 4, **dadurch gekennzeichnet, daß** eine Diffusorscheibe (40) vor dem Lichtsensor (10) angeordnet ist, die aus dem optischen Ausgang (8) der zu prüfenden Lichtleiteinrichtung (2) direkt auf den Lichtsensor (10) fallendes Licht zerstreut.

6. Vorrichtung nach einem der Ansprüche 1 bis 5, **dadurch gekennzeichnet, daß** die Lichtaustrittsachse des optischen Ausgangs (8) der zu prüfenden Lichtleiteinrichtung (2) durch die Öffnung (22) der Kammer (20) einen Winkel von ≤ 90° zu der Lichtdetektionsachse des Lichtsensors (10) aufweist.

7. Vorrichtung nach einem der Ansprüche 1 bis 6, **dadurch gekennzeichnet, daß** der Lichtsensor (10) aus einer Photodiode besteht.

8. Vorrichtung nach dem einem der Ansprüche 1 bis 7, **dadurch gekennzeichnet, daß** in der Auswerteeinrichtung (14) ein Referenzwert für die Lichtintensität der Lichtquelle (6) gespeichert ist und daß die Auswerteeinrichtung (14) den Transmissionsverlust als Quotienten des von dem Lichtsensor (10) gemessene Lichtintensitätswertes und dem Referenzwert berechnet und über die Anzeigeeinrichtung (18) als Prozentwert anzeigt.

9. Vorrichtung nach Anspruch 8, **dadurch gekennzeichnet, daß** der Referenzwert durch direkten Anschluß der Lichtquelle (6) an die Öffnung (22) der Kammer (20) und Messung der Referenzlichtintensität in die Auswerteeinrichtung (14) einspeicherbar ist.

10. Vorrichtung nach einem der Ansprüche 8 oder 9, **dadurch gekennzeichnet, daß** für vorbestimmte zu prüfende endoskopische Lichtleiteinrichtungen (2) Mindesttransmissionswerte in der Auswerteeinrichtung (14) gespeichert sind, die bei Durchführung der Messung als Referenzwerte abrufbar sind.

11. Vorrichtung nach einem der Ansprüche 1 bis 10, **dadurch gekennzeichnet, daß** die Kammer (20) in einer Hohlkugel (36) angeordnet ist.

## Claims

1. Device for detecting transmission losses of optical light guide means (2) by means of measurements, comprising optical light guide means (2) to be checked having an optical inlet portion (4) through which light from a light source (6) is transmitted to an optical outlet portion (8), at least one light sensor (10) arranged in a chamber (20) for measuring the light intensity emerging from the optical outlet portion (8) of the light guide means (2) to be checked and diffusely reflected in the chamber (20), and an evaluation means (14) for the measuring signal from the at least one light sensor (10), the evaluation means (14) having a display means (18) for displaying the measurement result,
**characterized in that**
said light guide means to be checked consists of an endoscope,
said optical inlet portion (4) of said light guide means (2) to be checked is located at the inlet side of the light guide means (2) contained in the endoscope (3),
said optical outlet portion (8) of said endoscope (3) is introduced through an opening (22) of the chamber (20) into the chamber (20), and
a separate light source (6) independent of the light guide means (2) to be checked contained in said endoscope (3) couples a predetermined light intensity into the optical inlet portion (4) of said endoscope (3).

2. Device according to claim 1, **characterized in that** the light source (6) consists of a light-emitting diode.

3. Device according to one of claims 1 to 2, **characterized in that** the light source (6) is arranged in a connector (28) which is adapted to the connection (32) of the optical inlet portion of the light guide means (2) to be checked.

4. Device according to one of claims 1 to 3, **characterized in that** the chamber (20) comprises a spherical inner surface (24).

5. Device according to one of claims 1 to 4, **characterized in that** a diffusion disk (40) is arranged in front of the light sensor (10), which diffusion disk (40) scatters the light from the optical outlet portion (8) of the light guide means (2) to be checked which directly impinges onto the light sensor (10).

6. Device according to one of claims 1 to 5, **characterized in that** the axis of the light emerging from the optical outlet portion (8) of the light guide means (2) to be checked through the opening (22) of the chamber (20) has an angle of ≤ 90° relative to the light detection axis of the light sensor (10).

7. Device according to one of claims 1 to 6, **characterized in that** the light sensor (10) consists of a photodiode.

8. Device according to one of claims 1 to 7, **characterized in that** a reference value for the light intensity of the light source (6) is stored in the evaluation means (14), and the evaluation device (14) calculates the transmission loss as the quotient of the light intensity value measured by the light sensor (10) and the reference value and displays it as a percentage value on the display means (18).

9. Device according to claim 8, **characterized in that** the reference value is adapted to be read into the evaluation means (14) by directly connecting the light source (6) to the opening (22) of the chamber (20) and measuring the reference light intensity.

10. Device according to one of claims 8 or 9, **characterized in that** for predetermined endoscopic light guide means (2) to be checked, minimum transmission values are stored in the evaluation means (14), which minimum transmission values are adapted to be recalled as reference values during the measuring process.

11. Device according to one of claims 1 to 10, **characterized in that** the chamber (20) is arranged in a hollow sphere (36).

## Revendications

1. Dispositif pour la saisie métrologique de pertes de transmission de dispositifs à fibre optique (2), avec une entrée optique (4) du dispositif à fibre optique (2) à vérifier qui transmet la lumière émise par une source lumineuse (6) vers une sortie optique (8), comportant au moins un capteur de lumière (10) qui est disposé dans une chambre (20) et qui mesure l'intensité de la lumière sortant par la sortie optique (8) du dispositif à fibre optique (2) à vérifier et réfléchie de façon diffuse dans la chambre (20) et un dispositif d'exploitation (14) qui exploite le signal de mesure de l'au moins un capteur de lumière (10) et qui comporte un dispositif d'affichage (18) destiné à afficher le résultat de la mesure,
**caractérisé en ce que** :
le dispositif à fibre optique à vérifier est constitué par un endoscope,
l'entrée optique (4) du dispositif à fibre optique (2) à vérifier se trouve du côté de l'entrée du dispositif à fibre optique (2) contenu dans l'endoscope (3),
la sortie optique (8) de l'endoscope (3) peut être insérée dans la chambre (20) par une ouverture (22) de la chambre (20), et
une source lumineuse séparée (6), indépendante du dispositif à fibre optique (2) à vérifier et se trouvant dans l'endoscope, injecte une intensité lumineuse prédéterminée dans l'entrée optique (4) de l'endoscope (3).

2. Dispositif selon la revendication 1, **caractérisé en ce que** la source lumineuse (6) est constituée par une diode luminescente.

3. Dispositif selon l'une des revendications 1 à 2, **caractérisé en ce que** la source lumineuse (6) est disposée dans une pièce de raccordement (28) qui est adaptée au raccord (32) de l'entrée optique du dispositif à fibre optique (2) à vérifier.

4. Dispositif selon l'une des revendications 1 à 3, **caractérisé en ce que** la chambre (20) présente une surface intérieure sphérique (24).

5. Dispositif selon l'une des revendications 1 à 4, **caractérisé en ce qu'**un disque diffuseur (40) est disposé en avant du capteur de lumière (10) et diffuse la lumière provenant de la sortie optique (8) du dispositif à fibre optique (2) à vérifier, directement incidente sur le capteur de lumière (10).

6. Dispositif selon l'une des revendications 1 à 5, **caractérisé en ce que** l'axe de sortie de lumière de la sortie optique (8) du dispositif à fibre optique (2) à vérifier, passant par l'ouverture (22) de la chambre (20), fait un angle de ≤ 90° par rapport à l'axe de détection de lumière du capteur de lumière (10).

7. Dispositif selon l'une des revendications 1 à 6, **caractérisé en ce que** le capteur de lumière (10) est formé par une photodiode.

8. Dispositif selon l'une des revendications 1 à 7, **caractérisé en ce qu'**une valeur de référence de l'intensité lumineuse de la source lumineuse (6) est mémorisée dans le dispositif d'exploitation (14) et **en ce que** le dispositif d'exploitation (14) calcule la perte de transmission sous la forme d'un quotient de la valeur d'intensité lumineuse mesurée par le capteur de lumière (10) et de la valeur de référence et l'affiche sous la forme d'un pourcentage sur le dispositif d'affichage (18).

9. Dispositif selon la revendication 8, **caractérisé en ce que** la valeur de référence peut être mémorisée dans le dispositif d'exploitation (14) en raccordant directement la source lumineuse (6) à l'ouverture (22) de la chambre (20) et en mesurant l'intensité de lumière de référence.

10. Dispositif selon l'une des revendications 8 ou 9, **caractérisé en ce que**, pour des dispositifs endoscopiques à fibre optique prédéterminés (2) à vérifier, des valeurs de transmission minimales sont mémorisées dans le dispositif d'exploitation (14), qui peuvent être appelées comme valeurs de référence lors de la mesure.

11. Dispositif selon l'une des revendications 1 à 10, **caractérisé en ce que** la chambre (20) est placée dans une sphère creuse (36).
